# EUROPEAN PATENT SPECIFICATION

(11) **EP 2 822 547 B1**
(45) Date of publication and mention of the grant of the patent: **08.08.2018**
(21) Application number: 13757518.9
(22) Date of filing: 05.03.2013
(51) Int. Cl.: A61K 31/132, A61K 31/167, A61K 45/06, A61P 15/02, A61P 43/00

(54) **SPERMIDINE OR SPERMINE FOR THE TREATMENT OF VULVAR VESTIBULAR SYNDROME**
SPERMIDIN ODER SPERMIN FÜR DIE BEHANDLUNG VON VESTIBULITIS VULVAE SYNDROM
SPERMIDINE OU SPERMINE POUR LE TRAITEMENT DU SYNDROME DES VESTIBULITES VULVAIRES

(30) Priority: 08.03.2012 IT MI20120363
(43) Date of publication of application: 14.01.2015
(73) Proprietor: Tixupharma, 20133 Milano (IT)
(72) Inventor: GHISALBERTI, Carlo, 04542-080 Sao Paulo (BR)
(74) Representative: Gislon, Gabriele
(86) International application number: PCT/IB2013/000524
(87) International publication number: WO 2013/132322

(56) References cited:
- WO-A1-2012/017290
- WO-A1-2012/017290
- WO-A2-99/51213
- WO-A2-99/51213
- WO-A2-2012/017288
- WO-A2-2012/017288
- FARAGE M A ET AL: "Vulvar vestibulitis syndrome: A review", EUROPEAN JOURNAL OF OBSTETRICS & GYNECOLOGY AND REPRODUCTIVEBIOLOGY, EXCERPTA MEDICA, AMSTERDAM, NL, vol. 123, no. 1, 1 November 2005 (2005-11-01), pages 9-16, XP027604504, ISSN: 0301-2115 [retrieved on 2005-11-01]

## Description

### FIELD OF THE INVENTION

The invention refers to a gynecologic local composition comprising spermidine and/or spermine to treat vestibulodynia, best known as vulvar vestibulitis syndrome.

### BACKGROUND

Vulvar vestibulitis syndrome (VVS) is one of the most common causes of genital pain and pain with sexual intercourse, affecting up to 15% of women in fertile age.

This syndrome is characterized by severe pain on vestibular touch or attempted vaginal entry, exquisite tenderness to palpation with a cotton swab, as well as by subtle physical findings confined to vestibular erythema. The etiology of VVS is unknown. Hence, it is probably best to consider VVS as a chronic local inflammatory condition with a wide variety of etiologic causes.

The heterogeneity of possible causative factors in VVS results in multiple treatment regimens into clinical practice (Goldstein AT et al., Vulvodynia: strategies for treatment; Clin Obstet Gynecol. 2005;48(4):769-85). More common prescription refers to local anaesthetics and systemic antidepressant, the latter maybe also for an ancillary inhibitory effect on mast cells degranulation.

Innovation under study includes nifedipine and enoxaparin to improve microvascularity; or botulin, capsaicin, pregabalin, and gabapentin with the aim of reducing the sensibility in the affected tissues.

Scientifically rigorous studies are sorely needed to determine the best approach in VVS. In this frame we have tentatively tested spermidine in sustained delivery system form, which proven effective in in vitro models, as disclosed by co-pending application WO12/017288 and WO12/017290. This therapeutic approach was conceived and is under testing in women's pathologies of dominant atrophic and senescent nature, such as in vaginal atrophy (VA).

While the reparative action performed by spermidine was expectedly beneficial in VA, no response could be initially expected in VVS, a predominantly inflammatory condition with a suspected underlying neurogenic component.

In fact, previous researches disclosed the spermidine pro-inflammaory action. Silva MA in "Role of peripheral polyamines in the development of inflammatory pain" (Biochem Pharmacol. 2011;82(3):269-77) suggested a contribution of Spd and PA in nociception and edema. In this study a local increase in inflammatory sites did correlate to an increase of precursor output, seemingly stimulated by PKC activation. Authors finally stated that limiting PA and Spd level and action could be a method of controlling inflammatory pain.

### SUMMARY

It was surprisingly discovered that spermidine represents an effective medical treatment in vulvar vestibulitis syndrome (VVS).

In an aspect, the invention refers to a local gynecologic composition for the treatment of WS comprising spermidine, salt, and supramolecular complex thereof.

In another aspect, the invention refers to a local composition for the treatment of VVS comprising spermidine from about 0.1% to 0.001% w/v of the composition.

In a further aspect, the invention refers to the afore-mentioned inventive composition comprising spermidine in combination or in conjuction with one or more anesthetic agent and an estrogenic substance in suitable concentration.

In a yet further aspect, the invention refers to the mentioned inventive composition wherein spermidine is partially or fully substituted with spermine.

These and other features of the invention are best described herein after.

### BRIEF DESCRIPTION OF THE DRAWING

FIG. 1 shows the self-evaluation (subjective score, SS) fby weekly questionnaire of a subject with VVS under a 4+4 weeks (W0-W8) treatment protocol with spermidine.
FIG. 2 shows the clinical evaluation (objective score, OS) at baseline (VI), by a next visit after 4 weeks (V2), and by the final visit after the further 4 weeks of treatment.(V3).

### DETAILED DESCRIPTION

The present invention refers to a medicinal, local composition comprising spermidine for the treatment of a gynecologic disorder with prevailing inflammatory and neurogenic signs named Vulvar Vestibulitis Syndrome (VVS).

The active ingredient of the present invention is spermidine, a substance of structure NH₂(CH₂)₃NH(CH₂)₄NH₂, and formula C₇H₁₉N₃.

Spermidine is easily incorporated as salt or complex in water-based gynecologic compositions; or as pure amine in water-free or emulsified gynecologic compositions.

Example of salt form are formed by spermidine with inorganic acid such as HCl, H₂SO₄, H₃BO₃, H₃PO₄, or with organic acid such as acetic, methylsulfonic, ascorbic, lauric, glycolic, lactic, pyruvic, succinic, and citric acid. In case of use of spermidine pure amine, partial or full anion exchange may take place *in situ* in contact with acidic substances in composition. Analogously, a simple salt such as spermidine 3HCl may undergo acid-base exchange within the composition medium.

In a further embodiment, the composition of invention will comprise a supramolecular complex with spermidine for the sustained release thereof.

The expression "supramolecular complex" as used herein includes polyacid complex formed by polyanionic polymer(s) and spermidine as well as the inclusion complex of spermidine into cyclodextrins. Technical and operative details are enclosed in co-pending applications WO12/017288 and WO12/017290.

The main inventive object is a topical medicinal composition for treating VVS, wherein "medicinal composition" include drugs, medical devices, and lenitive cosmetics intended for the specific curative purpose.

The inventive composition will comprise spermidine in concentration from about 1% to 0.0001% w/v, more preferably from about 0.1% to 0.001% w/v, even more preferably around 0.01% w/v of the composition.

In the inventive composition, the unitary dose of spermidine will be comprised between 1 mmoles and 1 nmoles/unit dose, preferably between 100 µmoles e 10 nmoles/unit dose, even more preferably around 10 µmoles and 100 nmoles/unit dosea.

The compositions according to the invention, which are administered vaginally, will be presented in semi-fluid forms such as of gel, cream, ointment, etc., or in solid form such as tablets, capsule, pessaries, ovules, etc. for the release of around 1 µmole of spermidine.

Excipients and auxiliary active ingredients to produce a formulation with proper safety, efficacy, patient compliance, aesthetics, acceptability to regulatory authorities, and cost requires are known. E.g., Garg S et al. in Compendium of Pharmaceutical Excipients for Vaginal Formulations Pharmaceutical Technology, Drug Delivery 2001, 14-24; listed vaginal excipients, the functional classification, allowed concentrations (when available), and regulatory status.

The inventive composition may be packaged in ordinary Al or plastic tubes for hand application; or as spray, mousse and other means for the application onto female external genitalia without a direct contact with hand, or other device. In general, no vaginal applicator is needed, unless burdens were widely spread out into the vaginal tract.

In another embodiment, compositions of invention comprise spermidine in combination or in conjunction with an anethetic agent to deliver a fast relief from VVS pain.

Exemplary anesthetic agents include ester-type like benzocaine, chlorprocaine, cocaine, procaine, tetracaine; and amide-type like lidocaine, prilocaine, mepivacaine, bupivacaine, ropivacaine. For use in combination, spermidine and anesthetic agent may be presented in ratio consistent with the desired effect. In particular, the molar ratio of spermidine to anesthetic agent will suitably be approximately 1 to 300. Preferably, this ratio will be between 0.001 to 3 and 1 to 3, and especially from 0.01:3 to 0.1:3.

In another embodiment, compositions of invention comprise spermidine in combination or in conjunction with an estrogenic substance to further improve the therapeutic ratio.

Exemplary estrogenic substances include estrone, estrone esters, estriol esters, estriol, equilin, equilin esters, estradiene, equilenin, ethinyl estradiol, 17β-estradiol, 17β-estradiol esters (i.e. benzoate, cypionate, dienanthate, valerate, etc.), 17α-dihydroequilenin, 17β-dihydroequilenin, 17α-dihydroequilin, 17p-dihydroequilin, 17α-ethynylestradiol, 17α-ethynylestradiol esters, dienestrol, mestranol, mestranol esters, DES, phytoestrogen(s), tibolone, ethynediol and conjugated estrogens.

Conjugated estrogens refer to estrogenic steroidal substances in which one or more functional groups (typically the OH groups) on the steroid exists as a conjugate (typically a sulfate or glucuronide). The conjugated estrogens may be a single conjugated estrogen, or may consist of mixtures of various conjugated estrogens.

For administration in combination, spermidine and the estrogenic substance may be presented in a ratio consistent with the desired effect. In particular, the molar ratio of spermidine to estrogenic substance will suitably be approximately 1 to 1. Preferably, this ratio will be between 0.01 to 1 and 100 to 1, and especially from 0.1:1 to 10:1.

The inventive composition may include other additional active ingredients. Examples include anti-infective agent such as antibiotics, anti-fungals, antivirals, biocides; calcium antagonists such as nifedipine; heparins such as enoxaparin; botulin; gabaergics such as pregabalin, and gabapentin anti-inflammatories; immuno-suppressant; plant or algal extracts; antihistamines; antioxidants; and a variety of other ingredients such as astringents, fragrances, dyes, vitamins, sunscreens, deodorants, preservatives, and other customary ingredients.

In another embodiment, spermidine is fully or partially replaced by the PA spermine, a substance of structure NH₂(CH₂)₃NH(CH₂)₄NH(CH₂)₃NH₂ and formula C₁₀H₂₆N₄.

### EXAMPLES

### EXAMPLE 1 - Test formulation

An investigational device (ID) for local use was prepared in form of gel having ingredients as set forth below.

| Ingredient | amount per 100 ml |
|---|---|
| Spermidine HCl | 10 mg |
| Sodium hyaluronate | 2.0 g |
| Sodium benzoate | 0.3 g |
| Benzyl alcohol | 0.3 g |
| Phenoxyethanol | 0.5 g |
| Citric acid | q.b. to pH 5.35 |
| Purified water | q.b. to 100 ml |

The resulting transparent gel was packaged in 30 ml Al tube in a GMP/GLP facility.

### EXAMPLE 1 - Pilot Study on VVS

The general design of clinical protocol is illustrated herein after, with a summary of the key protocol chapter given in brief.

### Primary Outcome Measures: Efficavy

• Change in physical signs of vestibulitis on physical exam after 4+4 weeks of treatment by gynaecology visit ranked with a vulvoVaginal Health Index (vVHI), whose 1-to-5 score is illustrated in Table I

**TABLE I - vVHI by Objective Score (OS)**

| SCORE | 1 | 2 | 3 | 4 | 5 |
|---|---|---|---|---|---|
| Vulvar inflammation (redness) | Vivid red with rashes | Very red and inflammed | Intermediate, minor spots | Mostly rose-colored | Rosy and healthy |
| Musculature contraction | Highly overal contracted | Anus or pelvis contracted | Intermediate | Fair-to-good | Excellent, fully relaxed |
| Pain at speculum insertion | Very strong | Strong | Moderate | Minimal | None |
| Epithelial integrity (mucosa) | Petechiae, ulcerate mucosa | Bleeds with ligh contact | Not friable, thin epithelium | Almost fully trophic | Normal |

• Change in self-reported, subjective symptoms (SS) of VVS from baseline to 4+4 weeks by weekly questionnaire, whose 0-to-3 score is illustrated in Table II.

**TABLE II - Subjective score (SS)**

| SCORE | 0 | 1 | 2 | 3 |
|---|---|---|---|---|
| Pruritus (Hitching) | None | Mild | Moderate | Severe |
| Burning | None | Mild | Moderate | Severe |
| Dryness | None | Mild | Moderate | Severe |
| Dyspareunia | None | Mildt | Moderate | Severe |

| | | | | |
|---|---|---|---|---|
| Meaning 0 = none (no symptoms during 24 hours); 1 = light (tolerable symptoms in few, short episode during 24 hours); 2 = moderate (tolerable symptoms for most 24 hours, with limited impairment of daily tasks); 3 = severe (hardly tolerable symptoms for most 24 hours, with relevant impairment of daily tasks). | | | | |

### Secondary endpoint: Tolerability and safety.

Tolerability, scored from excellent to poor, is included on patient's questionnaire. Safety was monitored from the adverse events (AE) report, that were recorded at all visits from subject experience or by physician controlled, to be reported and described in full length referring to date of beginning, intensity and duration.

### Inclusion criteria

Main inclusion criteria consist of:
- signature of the informed consensus ;
- women complaining a typical VVS, thereby confirmed by a local gynaecologic visit;
- collaborative subjects who are able to follow instruction and protocol schedule.

### Exclusion criteria

- subjects unable to supply a valid informed consensus;
- subjects using estrogens, or combined estrogens and progestinics;
- presence of autoimmune diseases, allergies, diabetes and hypertension;
- presence di cancer or chronic disease with less-then 2 year of life expectancy;
- therapy with corticosteroids or immuno-modulators within the past 3 weeks;
- previous or concomitant vulvovaginal laser therapy o crio-therapy;
- subjects unable to follow the protocol or comprehend scopes and effects;
- subjects under whatever experimental drug within the past 8 weeks.

### General design of the study

Visit 1 (V1, week 0): Enrolment with baseline assessment, ID delivery and instruction to start treatment by 3 applications per week;
Visit 2 (V2, week 4): After 4 weeks of treatment, objective assessment and next ID delivery for a new cycle with indication of two applications per week;
Visit 3 (V3, week 8): End of 4+4 weeks of treatment with final objective assessment and delivery of the questionnaire.

### Doses and treatment regimen

IP was delivered at V1 and V2 with instruction to apply on vulvar area by hand 3 times per week during the first 4 weeks; followed by 3 times per week during next 4 weeks.

### EXAMPLE 3 - Typical case report

A Caucasian woman aged 38 presented to the ambulatory asking medication for recalcitrant VVS. Beside that, health conditions were good, with no sign of allergy, sensitization, nor immuno-reactivity. She was primipara with regular menstrual cycles.

History Vulvodynia started 5 years before. Past attempts to treat VVS were various with no significant results. She furthermore reported recurrent vulvovaginitis from candidosis. Patient performed a pap test within past 6 month with negative response. She accepted to enter the pilot study after signing the informed consensus.

First visit (V1) Upon external exploration, vulva displayed reddish vulvar vestibulus as well as redness on inner labia minora. By inserting speculum, patient experienced strong pain and contracted perineum muscles. Instead, inner vaginal mucosa appeared normal. On SS, patient scored a fair vaginal burning, moderate itching and severe dyspareunia.

Second visit (V2) After 4 weeks of treatment by 3 weekly applications the patient referred about the increase of itching and burning by week6, then treated with local antifungal. Mucosa looked pink with reduced reddish areas. Speculum insert provoked less pain. Patient avoided sexual intercourses, hence dyspareunia was scored as unchanged.

Third visit (V3) After second cycle of 4 weeks by 2 weekly applications, examination revealed an almost normalized vulvar mucosa. Patient did not complain nor perceive pain at speculum insertion. During this period an episode of candidosis were medicated with local antibiotic therapy. Dyspareunia was scored as moderate.

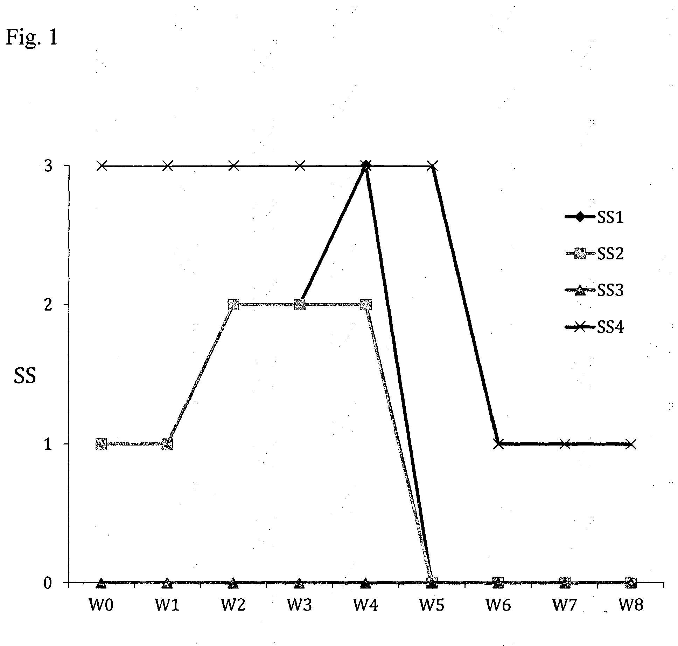

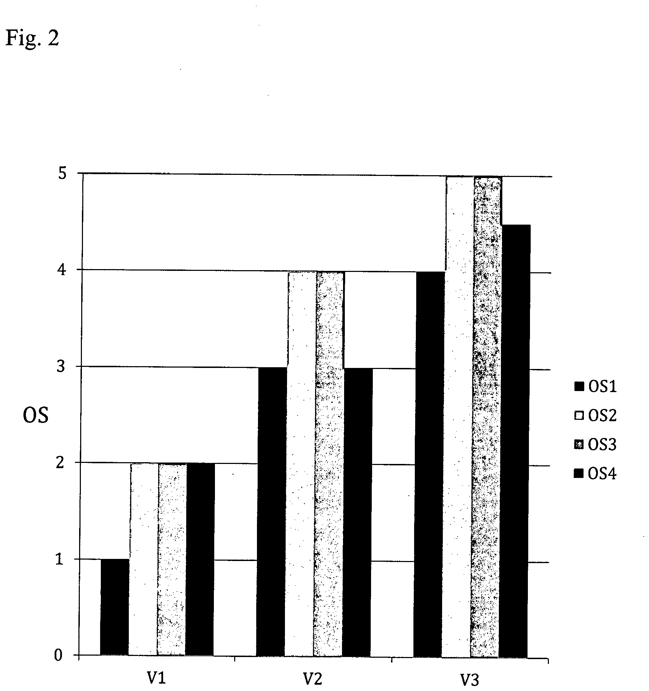

Conclusion Clinical remission was finally attained, as denoted by objective scores (SS) and objective scores (OS) trends, which are plotted in Fig. 1 and Fig. 2, respectively Interestingly, the improvement already found at visit 2 (V2) by objective examination did not correspond to the perceived symptoms, which persist or even worsened (beside the transient burdens due to candidosis in W4-W5) until a sudden improvement noticed from W6 on. Nonetheless, the two scores converged at the end of the treatment

### EXAMPLE 4 - Combination cream with anesthetic

An emulsion was prepared by mixing under turbo-emulsifier the oily phase melted at around 70°C with the water-soluble warmed at the same temperature, which end up with a cream having ingredients as set forth below.

| Ingredient | amount per 100 ml |
|---|---|
| Oily phase | |
| Cetyl alcohol | 5.0 g |
| Glyceryl stearate | 2.5 g |
| PEG100 stearate | 2.5 g |
| Dicaprylyl ether | 5.0 g |
| Cyclopentaxiloxane | 5.0 g |
| Lidocaine (base) | 2.0 g |

| Water phase | |
|---|---|
| Spermidine HCl | 10 mg |
| Methylisoxazolidone ,10% sol. | 0.1 g |
| Benzyl alcohol | 0.3 g |
| Purified water | q.b. to 100 ml |

The resulting cream was packaged in 30 ml Al tube under GMP/GLP provisions. It provides an immediate pain relief from the very beginning of the spermidine therapy.

### EXAMPLE 5 - Combination gel with anesthetic

The emulsion obtained in Example 1 was modified by adding 10 mg g of b-estradiol instead of 2 g of lidocaine.

The innovation entails the local use of spermidine to treat VVS.

## Claims

1. A local gynecologic composition comprising spermidine for use in treatment of vulvar vestibulitis syndrome (VVS).

2. The composition for use according to claim 1 wherein spermidine is present as addition salt in concentration between 100 µmoles and 10 nmoles per unit dose.

3. The composition for use according to claim 1 wherein spermidine is present within a supramolecular complex in concentration between 100 µmoles and 10 nmoles per unit dose.

4. The composition for use according to claim 2 or 3 wherein spermidine is present in concentration between 10 and 0.1 µmoles per unit dose along with physiologically acceptable excipients.

5. The composition for use according to any of claims 1-4 in the form of gel, cream, ovule, solution, tablets, or other compositions suitable for the local release on vulva.

6. The composition for use according to any of claims from 1 to 5 further comprising an additional active ingredient.

7. The composition for use according to claim 6 wherein said additional active ingredient is an anesthetic agent.

8. The composition for use according to claim 6 wherein said additional active ingredient is an estrogenic substance.

9. The composition for use according to any of the previous claims wherein spermidine is present in concentration from about 1% to 0.0001% w/v, more preferably from about 0.1% to 0.001% w/v.

10. The composition for use according to any of the previous claims wherein spermidine is partially or fully replaced by spermine.

11. Spermidine for use in treatment of vulvar vestibulitis syndrome (VVS).

12. Spermidine for use according to claim 11, in combination with an additional active ingredient.

13. A composition for use according to claim 5, in the form of a gel having ingredients as set forth below:
| Ingredient | amount per 100 ml |
|---|---|
| Spermidine HCl | 10 mg |
| Sodium hyaluronate | 2.0 g |
| Sodium benzoate | 0.3 g |
| Benzyl alcohol | 0.3 g |
| Phenoxyethanol | 0.5 g |
| Citric acid | q.b. to pH 5.35 |
| Purified water | q.b. to 100 ml |

## Patentansprüche

1. Lokale gynäkologische Zusammensetzung enthaltend Spermidin zur Verwendung in der Behandlung von vulvärem Vestibulitis-Syndrom (VVS).

2. Zusammensetzung zur Verwendung nach Anspruch 1, wobei Spermidin als Additionssalz in einer Konzentration zwischen 100 µmol und 10 nmol pro Einheitsdosis vorhanden ist.

3. Zusammensetzung zur Verwendung nach Anspruch 1, wobei Spermidin in einem supramolekularen Komplex in einer Konzentration zwischen 100 µmol und 10 nmol pro Einheitsdosis vorhanden ist.

4. Zusammensetzung zur Verwendung nach Anspruch 2 oder 3, wobei Spermidin in einer Konzentration zwischen 10 und 0,1 µmol pro Einheitsdosis mit physiologisch annehmbaren Exzipienten vorhanden ist.

5. Zusammensetzung zur Verwendung nach einem der Ansprüche 1 bis 4 in der Form eines Gels, einer Creme, eines Ovulums, einer Lösung, von Tabletten oder anderer Zusammensetzungen, die für die lokale Freisetzung auf Vulva geeignet sind.

6. Zusammensetzung zur Verwendung nach einem der Ansprüche 1 bis 5, weiterhin umfassend einen zusätzlichen Wirkstoff.

7. Zusammensetzung zur Verwendung nach Anspruch 6, wobei der zusätzliche Wirkstoff ein Anästhetikum ist.

8. Zusammensetzung zur Verwendung nach Anspruch 6, wobei der zusätzliche Wirkstoff eine Östrogensubstanz ist.

9. Zusammensetzung zur Verwendung nach einem der vorherigen Ansprüche, wobei Spermidin in einer Massenkonzentration (Gew./Vol.) von ungefähr 1 % bis 0,0001 %, vorzugsweise von ungefähr 0,1 % bis 0,001 % vorhanden ist.

10. Zusammensetzung zur Verwendung nach einem der vorherigen Ansprüche, wobei Spermidin teilweise oder vollständig durch Spermin ersetzt ist.

11. Spermidin zur Verwendung in der Behandlung von vulvärem Vestibulitis-Syndrom (VVS).

12. Spermidin zur Verwendung nach Anspruch 11 in Kombination mit einem zusätzlichen Wirkstoff.

13. Zusammensetzung zur Verwendung nach Anspruch 5 in der Form eines Gels mit Inhaltsstoffen wie unten definiert:
| Bestandteil | Menge pro 100 ml |
|---|---|
| Spermidin HCl | 10 mg |
| Natriumhyaluronat | 2,0 g |
| Natriumbenzoat | 0,3 g |
| Benzylalkohol | 0,3 g |
| Phenoxyethanol | 0,5 g |
| Zitronensäure | soviel wie nötig bis pH 5,35 |
| demineralisiertes Wasser | soviel wie nötig bis 100 ml. |

## Revendications

1. Composition gynécologique locale comprenant de la spermidine pour son utilisation dans le traitement du syndrome de la vestibulite vulvaire (SVV).

2. Composition pour utilisation selon la revendication 1, dans laquelle la spermidine est présente en tant que sel d'addition en une concentration entre 100 µmoles et 10 nmoles par dose unitaire.

3. Composition pour utilisation selon la revendication 1, dans laquelle la spermidine est présente dans un complexe supramoléculaire en une concentration entre 100 µmoles et 10 nmoles par dose unitaire.

4. Composition pour utilisation selon la revendication 2 ou 3, dans laquelle la spermidine est présente en une concentration entre 10 et 0,1 µmoles par dose unitaire conjointement à des excipients physiologiquement acceptables.

5. Composition pour utilisation selon l'une quelconque des revendication 1 à 4, sous la forme d'un gel, d'une crème, d'un ovule, d'une solution, de comprimés ou autres compositions appropriées pour la libération locale sur la vulve.

6. Composition pour utilisation selon l'une quelconque des revendications 1 à 5, comprenant en outre un ingrédient actif supplémentaire.

7. Composition pour utilisation selon la revendication 6, dans laquelle ledit ingrédient actif supplémentaire est un agent anesthésique.

8. Composition pour utilisation selon la revendication 6, dans laquelle ledit ingrédient actif supplémentaire est une substance oestrogénique.

9. Composition pour utilisation selon l'une quelconque des revendications précédentes, dans laquelle la spermidine est présente en une concentration d'environ 1 % à 0,0001 % p/v, de manière davantage préférée d'environ 0,1 % à 0,001 % p/v.

10. Composition pour utilisation selon l'une quelconque des revendications précédentes, dans laquelle la spermidine est partiellement ou complètement remplacée par la spermine.

11. Spermidine pour utilisation dans un traitement du syndrome de la vestibulite vulvaire (SVV).

12. Spermidine pour utilisation selon la revendication 11, en combinaison avec un ingrédient actif supplémentaire.

13. Composition pour utilisation selon la revendication 5, sous la forme d'un gel ayant des ingrédients tels que définis ci-dessous :
| Ingrédients | quantité pour 100 ml |
|---|---|
| Spermidine HCl | 10 mg |
| Hyaluronate de sodium | 2,0 g |
| Benzoate de sodium | 0,3 g |
| Alcool benzylique | 0,3 g |
| Phénoxyéthanol | 0,5 g |
| Acide citrique | q.s. pour pH 5,35 |
| Eau purifiée | q.s. pour 100 ml |
